# Europäisches Patentamt

# European Patent Office

# Office européen des brevets

(19)

(11) Veröffentlichungsnummer: **0 224 220 B1**

(12) **EUROPÄISCHE PATENTSCHRIFT**

(45) Veröffentlichungstag der Patentschrift:
22.02.89

(21) Anmeldenummer: 86116189.1

(22) Anmeldetag: **22.11.86**

(51) Int. Cl.⁴: **C07C 11/02, C07C 2/12**

(54) Verfahren zur Herstellung von Diisobuten aus Isobuten.

(30) Priorität: **29.11.85 DE 3542171**

(43) Veröffentlichungstag der Anmeldung:
03.06.87 Patentblatt 87/23

(45) Bekanntmachung des Hinweises auf die Patenterteilung:
22.02.89 Patentblatt 89/8

(84) Benannte Vertragsstaaten:
**BE CH DE FR GB IT LI NL**

(56) Entgegenhaltungen:
**EP-A- 0 007 081**
**DD-A- 154 983**

(73) Patentinhaber: **BASF Aktiengesellschaft,
Carl-Bosch-Strasse 38, D-6700 Ludwigshafen(DE)**

(72) Erfinder: **Hoelderich, Wolfgang, Dr., Mannheimer
Strasse 18c, D-6710 Frankenthal(DE)**
Erfinder: **Hoffmann, Herwig, Dr., Knietschstrasse 21,
D-6710 Frankenthal(DE)**
Erfinder: **Lermer, Helmut, Dr., D 3,4,
D-6800 Mannheim 1(DE)**

## Beschreibung

Diese Erfindung betrifft ein Verfahren zur Herstellung von Diisobuten, insbesondere von 2,4,4-Trimethylpenten-1 und -2, durch Umsetzung von Isobuten an einem Zeolith-Katalysator, der mit Wismut und/oder Blei dotiert ist, bei höherer Temperatur.

Die Oligomerisation bzw. Polymerisation von niederen Olefinen zu höher molekularen α-Olefinen ist von großem technischem Interesse. Diese α-Olefine werden unter anderem zur Alkylierung von Aromaten (z.B. Dodecen) für die Waschmittelindustrie eingesetzt oder dienen als synthetische Schmiermittel.

Im allgemeinen führt man die Oligomerisation der niederen Olefine an Phosphorsäure enthaltenden Katalysatoren oder an $BF_3$ als Katalysator durch und zwar meist zusammen mit Kokatalysatoren, wie Alkoholen ($BF_3$-Decanol), Säuren ($BF_3$-Essigsäure), Estern, Polyolen oder Ketonen.

Man hat die Oligomerisation von Olefinen auch schon an Zeolith-Katalysatoren durchgeführt. So werden z.B. nach den Angaben der US-PSen 3 756 942 und 3 827 968 und der GB-PS 1 489 646 Aluminosilikatzeolithe, wie ZSM 5 und ZSM 11, die teilweise mit $Al_2(SO_4)_3$ oder $CrCl_3$ promotiert sind, oder mit Ni- und Co-dotierte Zeolithe verwendet. Diese Verfahren sind auf die Aromatenherstellung ausgerichtet, die durch die stark aciden Aluminosilikatzeolithe begünstigt wird. Nach den Angaben der US-PS 4 021 502 verwendet man ebenfalls Aluminiumsilikatzeolithe, wie ZSM 4, ZSM 12, ZSM 18, Chabazit und Zeolith für die Oligomerisation von $C_2$–$C_5$-Olefinen zum Zwecke der Benzingewinnung. Zur Herstellung von synthetischen Schmierölen wird die Oligomerisation von $C_2$–$C_6$-Olefinen an Aluminiumoxid-freien Molekularsieben, sog. Silicalit oder CZM, die mit Zn oder Cd dotiert sein können, durchgeführt (DE-OS 3 229 895).

Die Dimerisierung von Isobuten an Zeolithen wird in der DD 154 983 und in der US-PS 3 325 465 beschrieben. Nach den Angaben der DD 154 983 wird ein mit Aluminiumalkoholaten modifizierter Zeolith eingesetzt. Bei Temperaturen von 20 bis 70°C beträgt die Ausbeute 65% Di-, 25% Tri- und 6% Tetraisobuten. In der US-PS 3 325 465 ist angegeben, daß Isobuten an einem mit Co dotierten Zeolith 13 X bei 73°C zu 7,5% dimerisiert mit einer Selektivität an Diisobutenisomeren von 99% (eine genaue Zusammensetzung ist nicht angegeben).

Die bekannten Verfahren der Oligomerisation von niederen Olefinen sind bevorzugt auf die Herstellung von Aromaten, Benzingemischen bzw. synthetischen Schmierölen ausgerichtet. Die Herstellung des dimeren bzw. trimeren des Isobutens nach DD 154 983 setzt eine arbeits- und kostenaufwendige Katalysatorpräparation mit Aluminiumalkoholaten voraus. Außerdem erhält man hierbei neben dem gewünschten Diisobuten verstärkt Tri- und Tetraisobuten. Bei der Dimerisierung nach der US-PS 3 325 465 wird zwar eine hohe Selektivität an Diisobutenisomeren erhalten. Man erhält aber eben nur ein Gemisch vieler isomerer Verbindungen, unter denen sich die gewünschten 2,4,4-Trimethylpentene ebenfalls befinden können, welche aber nicht als Hauptprodukt auftreten.

Es bestand deshalb die Aufgabe, nach einem Verfahren zu suchen, das es gestattet, Isobuten unter Vermeidung der genannten Nachteile mit hoher Selektivität an 2,4,4-Trimethylpenten-1 und -2 zu dimerisieren.

Nach dem erfindungsgemäßen Verfahren, durch das diese Aufgabe gelöst wird, stellt man Diisobuten durch Dimerisierung von Isobuten an einem Zeolith-Katalysator bei höherer Temperatur dadurch her, daß man einen Zeolith-Katalysator vom Pentasiltyp, der 0,5 bis 7 Gew.-%, Wismut und/oder Blei enthält, verwendet.

Nach dem Verfahren der Erfindung erhält man 2,4,4-Trimethylpenten-1 und -2 mit hoher Selektivität und bei hohen Standzeiten.

Zeolithe sind kristalline Aluminosilikate, die eine hochgeordnete Struktur mit einem starren dreidimensionalen Netzwerk von $SiO_4$- und $AlO_4$- Tetraedern besitzen, die durch gemeinsame Sauerstoffatome verbunden sind. Das Verhältnis der Si- und Al-Atome zu Sauerstoff beträgt 1:2. Die Elektrovalenz der Aluminium enthaltenden Tetraeder ist durch Einschluß von Kationen in den Kristall, z.B. eines Alkali- oder Wasserstoffions ausgeglichen. Ein Kationenaustausch ist möglich. Die Räume zwischen den Tetraedern sind vor der Dehydratation durch Trocknen bzw. Calcinieren von Wassermolekeln besetzt. In den Zeolithen können anstelle von Aluminium auch andere Elemente, wie B, Ga, Fe, Cr, V, As, Sb in das Gitter eingebaut werden. Das Silicium kann durch ein vierwertiges Element wie Ge ersetzt werden.

Als Katalysatoren verwendet man Zeolithe vom Pentasil-Typ. Diese Zeolithe, die vor ihrer erfindungsgemäßen Verwendung noch mit Wismut und/oder Blei modifiziert werden, können unterschiedliche chemische Zusammensetzung aufweisen. Es handelt sich hierbei um Alumino-, Boro-, Eisen-, Gallium-, Chrom-, Arsen- oder Antimonsilikatzeolithe oder deren Gemische. Auch gehören hierzu die isotaktischen Zeolithe, wie sie in der DE-OS 3 006 471 beschrieben werden.

Besonders gut eignen sich die Alumino-, Boro- und Eisensilikatzeolithe des Pentasiltyps für die erfindungsgemäße Dimerisierung. Der Aluminosilikatzeolith wird z.B. aus einer Aluminiumverbindung, vorzugsweise $Al(OH)_3$ oder $Al_2(SO_4)_3$ und einer Siliciumkomponente, vorzugsweise hochdispersem Siliciumdioxid, in wäßriger Aminlösung, insbesondere in 1,6-Hexandiamin- oder 1,3-Propandiamin- oder Triethylentetramin-Lösung, mit oder ohne Alkali- oder Erdalkalizusatz bei 100 bis 220°C unter autogenem Druck hergestellt. Die erhaltenen Aluminosilikatzeolithe weisen je nach Wahl der Einsatzstoffmengen ein $SiO_2/Al_2O_3$-Verhältnis von 10 bis 40 000 auf. Auch lassen sich derartige Aluminosilikatzeolithe in etherischem Medium, wie Diethylenglykoldimethylether, in alkoholischem Medium, wie Methanol oder 1,4-Butandiol oder aber in Wasser synthetisieren.

Der Borosilikatzeolith wird z.B. bei 90 bis 200°C unter autogenem Druck synthetisiert, indem man eine Borverbindung, wie $H_3BO_3$, mit einer Siliciumverbindung, vorzugsweise hochdispersem Siliciumdioxid, in wäßriger Aminlösung, insbesondere in 1,6-Hexandiamin- oder 1,3-Propandiamin- oder Triethylentetramin-Lösung, mit oder ohne Alkali- oder Erdalkalizusatz zur Reaktion bringt. Solche Borosilikatzeolithe können auch dadurch hergestellt werden, daß man die Reaktion statt in wäßriger Aminlösung in etherischer Lösung, z.B. Diethylenglykoldimethylether oder in alkoholischer Lösung, z.B. 1,6-Hexandiol, durchführt.

Den Eisensilikatzeolith erhält man z.B. aus einer Eisenverbindung, vorzugsweise $Fe_2(SO_4)_3$ und einer Siliciumverbindung, vorzugsweise hochdispersem Siliciumdioxid in wäßriger Aminlösung, insbesondere 1,6-Hexandiamin, mit oder ohne Alkali- oder Erdalkalizusatz bei 100 bis 220°C unter autogenem Druck.

Die so hergestellten Alumino-, Boro- und Eisensilikatzeolithe können nach ihrer Isolierung, Trocknung bei 100 bis 160°C, vorzugsweise 110°C und Calcinierung bei 450 bis 550°C, vorzugsweise 500°C, mit einem Bindemittel im Verhältnis 90:10 bis 40:60 Gew.-% zu Strängen oder Tabletten verformt werden. Als Bindemittel eignen sich diverse Aluminiumoxide, bevorzugt Boehmit, amorphe Aluminosilikate mit einem $SiO_2/Al_2O_3$-Verhältnis von 25:75 bis 90:5, bevorzugt 75:25, Siliciumdioxid, bevorzugt hochdisperses $SiO_2$, Gemische aus hochdispersem $SiO_2$ und hochdispersem $Al_2O_3$, hochdisperses $TiO_2$ sowie Ton. Nach der Verformung werden die Extrudate oder Preßlinge z.B. bei 110°C/16 Stunden getrocknet und z.B. bei 500°C/16 Stunden calciniert.

Man erhält auch vorteilhaft Katalysatoren, wenn der isolierte Alumino- bzw. Boro- bzw. Eisensilikatzeolith direkt nach der Trocknung verformt wird und erstmals nach der Verformung einer Calcinierung unterworfen wird. Die hergestellten Alumino-, Boro- und Eisensilikatzeolithe können aber auch in reiner Form, ohne Binder, als Stränge oder Tabletten eingesetzt werden, wobei als Verstrangungs- oder Peptisierungshilfsmittel z.B. Ethylcellulose, Stearinsäure, Kartoffelstärke, Ameisensäure, Oxalsäure, Essigsäure, Salpetersäure, Ammoniak, Amine, Silikoester und Graphit oder deren Gemische verwendet werden.

Liegt der Zeolith aufgrund der Art seiner Herstellung nicht in der aciden H-Form vor, sondern z.B. in der Na-Form, dann kann diese durch Ionenaustausch z.B. mit Ammoniumionen und anschließende Calcinierung oder durch Behandlung mit Säuren vollkommen oder partiell in die gewünschte H-Form überführt werden.

Der erfindungsgemäß zu verwendende Zeolith-Katalysator ist mit Wismut und/oder Blei modifiziert. Sein Wismut- oder Blei-Gehalt beträgt 0,5 bis 7, vorzugsweise 0,7 bis 3,5 Gew.-%. Die Dotierung des Zeoliths mit Wismut oder Blei zur Herstellung des benötigten Katalysators erfolgt z.B. durch Ionenaustausch oder durch Imprägnierung des unverformten oder verformten Zeolithen mit Wismut- oder Bleisalzen.

Zweckmäßig führt man die Dotierung so durch, daß man z.B. den verformten Zeolithen in einem Steigrohr vorlegt und bei 20 bis 100°C z.B. eine wäßrige oder alkoholische Lösung eines Halogenids oder eines Nitrats oder Acetats des Wismuts oder Bleis überleitet. Ein derartiger Ionenaustausch kann z.B. an der Wasserstoff-, Ammonium- und Alkaliform des Zeolithen vorgenommen werden. Eine weitere Möglichkeit der Metallaufbringung auf den Zeolithen ist die, daß man das zeolithische Material z.B. mit einem Halogenid, einem Acetat, einem Nitrat oder einem Oxid des Wismuts oder Bleis in wäßriger oder alkoholischer Lösung imprägniert. Sowohl an einen Ionenaustausch als auch an eine Imprägnierung schließt sich zumindest eine Trocknung, wahlweise eine abermalige Calcinierung an.

Eine mögliche Ausführungsform besteht z.B. darin, daß man $Bi(NO_3)_3$ oder $Pb(CH_3COO)_2$ in Wasser löst. Mit dieser Lösung wird der verformte oder unverformte Zeolith eine gewisse Zeit, ca. 30 Minuten, getränkt. Die eventuell überstehende Lösung wird am Rotationsverdampfer von Wasser befreit. Danach wird der getränkte Zeolith z.B. bei 150°C getrocknet und z.B. bei 550°C calciniert. Dieser Tränkvorgang kann mehrmals hintereinander vorgenommen werden, um den gewünschten Metallgehalt einzustellen. Auch ist es möglich, z.B. den reinen pulverförmigen Zeolithen in einer Bleisalz- oder Wismutsalz-Lösung bei 40 bis 100°C unter Rühren ca. 24 Stunden aufzuschlämmen. Nach Abfiltrieren, Trocknen, z.B. bei 150°C, und Calcinierung, z.B. bei 500°C, kann das so gewonnene zeolithische Material mit oder ohne Bindemittel zu Strängen, Pellets oder Wirbelgut weiterverarbeitet werden.

Ein Ionenaustausch des in der H-Form vorliegenden Zeolithen kann so vorgenommen werden, daß man den Zeolithen in Strängen oder Pellets in einer Kolonne vorlegt und darüber z.B. eine wäßrige $Bi(NO_3)_3$- oder $Pb(CH_3COO)_2$-Lösung bei leicht erhöhter Temperatur zwischen 30 und 80°C im Kreislauf 15 bis 20 Stunden leitet. Danach wird mit Wasser ausgewaschen, z.B. bei 150°C getrocknet und z.B. bei 550°C calciniert. Die Katalysatoren können wahlweise als 2 bis 4 mm Stränge oder als Tabletten mit 3 bis 5 mm Durchmesser oder als Pulver mit Teilchengrößen von 0,1 bis 0,5 mm oder als Wirbelkontakt eingesetzt werden.

Wenn bei der erfindungsgemäßen Verwendung der zeolithischen Katalysatoren eine durch Koksabscheidung bedingte Desaktivierung eintritt, empfiehlt es sich, die Zeolithe durch Abbrennen der Koksablagerung mit Luft oder mit einem Luft/$N_2$-Gemisch bei 400 bis 550°C, bevorzugt 500°C, zu regenerieren. Die Zeolithe erhalten dadurch ihre Anfangsaktivität zurück.

Durch partielle Verkokung (pre-coke) ist es möglich, die Aktivität des Katalysators für ein Selektivitätsoptimum des gewünschten Reaktionsproduktes einzustellen.

Die Dimerisierung des Isobutens zum Diisobuten führt man an den Zeolithen vorzugsweise in der Gasphase bei Temperaturen von 70 bis 300°C, vorzugsweise 100 bis 200°C, durch. Die Belastung (WHSV) beträgt 0,1 bis 20, bevorzugt 1 bis 6 kg Isobuten pro kg Katalysator und Stunde. Im allgemeinen steigt der Umsatz mit steigender Temperatur stark an, während die Selektivität für 2,4,4-Trimethylpentene in einen bestimmten Temperaturbereich nur wenig zurückgeht. Das Verfahren kann bei Normaldruck oder erhöhtem Druck durchgeführt werden, wobei die Durchführung vorzugsweise kontinuierlich erfolgt.

Nach der Umsetzung werden die entstandenen Diisobutene auf an sich übliche Weise, z.B. durch Destillation, aus dem Reaktionsgemisch isoliert. Nichtumgesetztes Isobuten wird für die erfindungsgemäße Umsetzung zurückgeführt.

Beispiele 1 bis 11

Isobuten wurde zum Zwecke der Dimerisierung zu Diisobuten unter isothermen Bedingungen in einem Rohrreaktor (Wendelform, Innendurchmesser 0,6 cm und Länge 90 cm) eingeführt und in der Gasphase über den Zeolith-Katalysator geführt. Das Isobuten wurde dabei aus einem Vorratsgefäß flüssig (5 bar Druck) über eine Pumpe gefördert. Nach der Pumpe wurde drucklos gearbeitet. Die Charakterisierung der Reaktionsprodukte erfolgte durch GC bzw. GC-MS.

Die quantitative Bestimmung der Reaktionsprodukte und der Ausgangsstoffe erfolgte gaschromatographisch. Die gewählte Temperatur, die Art des Katalysators, die Belastung (WHSV), der Umsatz und die Selektivität sind der folgenden Tabelle zu entnehmen.

| Beispiel | 1 | 2 | 3 | 4 | 5 | 6 | 7 | 8 | 9 | 10 | 11 |
|---|---|---|---|---|---|---|---|---|---|---|---|
| Katalysator | A | A | C | C | D | E | B | F | F | G | H |
| Temperatur | 150°C | 150°C | 100°C | 150°C | 150°C | 150°C | 150°C | 150°C | 200°C | 150°C | 150°C |
| WHSF | $5,4\,h^{-1}$ | $12,6\,h^{-1}$ | $4,9\,h^{-1}$ | $4,8\,h^{-1}$ | $5,7\,h^{-1}$ | $5,2\,h^{-1}$ | $5,0\,h^{-1}$ | $4,9\,h^{-1}$ | $3,9\,h^{-1}$ | $5,4\,h^{-1}$ | $4,5\,h^{-}$ |
| Umsatz % | 15,2 | 8,3 | 17,8 | 19,7 | 28,6 | 24,2 | 31,5 | 23,8 | 33,1 | 45,8 | 41,0 |
| Selektivität % | | | | | | | | | | | |
| 2,2,4-Trimethylpenten-1 | 48,5 | 55,9 | 56,6 | 51,6 | 44,0 | 46,5 | 28,3 | 57,2 | 38,1 | 45,6 | 44,8 |
| 2,2,4-Trimethylpenten-2 | 12,0 | 15,7 | 15,3 | 18,8 | 16,1 | 17,2 | 7,0 | 19,6 | 15,2 | 16,1 | 16,0 |
| 2,3,4-Trimethylpentene | 5,3 | 5,7 | 3,9 | 12,3 | 22,4 | 23,2 | 1,5 | 8,4 | 25,1 | 11,7 | 10,8 |
| $C_8$ | 67,2 | 79,2 | 77,7 | 87,8 | 85,6 | 86,9 | 37,1 | 87,9 | 86,5 | 73,6 | 72,4 |
| $C_{12}$ | 32,1 | 19,2 | 20,7 | 12,0 | 14,3 | 13,0 | 62,5 | 12,1 | 11,2 | 26,6 | 22,0 |

Die verwendeten Katalysatoren wurden folgendermaßen hergestellt:

Katalysator A (Vergleichskatalysator)

Man stellt einen Borzeolith des Pentasiltyps her, indem man ein Gemisch aus 640 g hochdispersem $SiO_2$, 122 g $H_3BO_3$, 8000 g einer wäßrigen 1,6-Hexandiamin-Lösung (Mischung 50:50 Gew.-%) unter autogenem Druck in einem Rührautoklaven auf 170°C erhitzt. Nach Abfiltrieren und Auswaschen wird das kristalline Reaktionsprodukt bei 100°C/24 Stunden getrocknet und bei 500°C/24 Stunden calciniert. Dieser Borosilikatzeolith setzt sich zusammen aus 94,2 Gew.-% $SiO_2$ und 2,3 Gew.-% $B_2O_3$. Aus diesem Zeolith werden durch Verformen mit Boehmit im Gewichtsverhältnis 60:40 2-mm-Stränge hergestellt, die bei 110°C/16 Stunden getrocknet und bei 500°C/24 Stunden calciniert werden.

Katalysator B (Vergleichskatalysator)

Ein Aluminosilikatzeolith vom Pentasil-Typ wird bei autogenem Druck und 150°C aus 650 g hochdispersem $SiO_2$, 203 g $Al_2(SO_4)_3$, 18 $H_2O$ in 10 kg einer wäßrigen 1,6-Hexandiamin-Lösung (Mischung 50:50 Gew.-%) in einem Rührautoklaven hergestellt. Nach Abfiltrieren und Auswaschen mit Wasser wird das kristalline Reaktionsprodukt bei 110°C/24 Stunden getrocknet und bei 500°C/24 Stunden calciniert. Der so hergestellte Aluminosilikatzeolith enthält 92,8 Gew.-% $SiO_2$ und 4,2 Gew.-% $Al_2O_3$. Dieser Zeolith wird mit einem Peptisierungshilfsmittel zu 2-mm-Strängen verformt. Dann wird er bei 119°C getrocknet und bei 500°C/16 Stunden calciniert.

Katalysator C

Man imprägniert Katalysator A so mit einer wäßrigen $Bi(NO_3)_3$-Lösung, daß der Katalysator nach anschließender Trocknung (130°C/2 Stunden) und Calcination (540°C/2 Stunden) einen Bi-Gehalt von 2,7 Gew.-% aufweist.

Katalysator D

Man verfährt wie bei der Herstellung des Katalysators C, wobei man jedoch den Bi-Gehalt auf 0,78 Gew.-% einstellt.

Katalysator E

100 g Katalysator A werden mit 8 g $Bi(NO_3)_3 \times 5\ H_2O$ gelöst. Man vermischt mit 5 ml $HNO_3$ (65%ig) und 58 ml $H_2O$. Überstehende wäßrige Lösung wird am Rotationsverdampfer entfernt. Der Rückstand wird bei 130°C/2 h getrocknet und bei 540°C/2 h calciniert. Der Bi-Gehalt des Katalysators E beträgt 3,15 Gew.-%.

Katalysator F

Man imprägniert Katalysator B so mit einer wäßrigen $Bi(NO_3)_3$-Lösung, daß er nach Trocknung bei 130°C/2 Stunden und Calcinierung bei 540°C/2 Stunden 3,0 Gew.-% Bi enthält.

Katalysator G

Man verfährt wie bei der Herstellung des Katalysators F, wobei man jedoch den Bi-Gehalt auf 1,7 Gew.-% einstellt.

Katalysator H

Man verfährt wie bei Katalysator B angegeben, wobei man den Aluminosilikatzeolith jedoch ohne Bindemittel mit einem Verstrangungshilfsmittel zu 2 mm Strängen verformt, bei 130°C/16 h trocknet und bei 540°C/16 h calciniert. Diese Stränge werden mit einer wäßrigen Lösung von $Pb(CH_3COO)_2 \times 3\ H_2O$ imprägniert. Die mit Pb dotierten Stränge werden danach bei 130°C/2 h getrocknet und bei 540°C/2 h calciniert. Der Pb-Gehalt des Katalysators beträgt 2,2 Gew.-%.

**Patentansprüche**

1. Verfahren zur Herstellung von Diisobuten durch Dimerisierung von Isobuten an einem Zeolith-Katalysator bei höherer Temperatur, dadurch gekennzeichnet, daß man einen Zeolith-Katalysator vom Pentasiltyp, der 0,5 bis 7 Gew.-% Wismut und/oder Blei enthält, verwendet.
2. Verfahren nach Anspruch 1, dadurch gekennzeichnet, daß man einen Aluminosilikatzeolith-Katalysator verwendet.
3. Verfahren nach Anspruch 1, dadurch gekennzeichnet, daß man einen Borosilikatzeolith-Katalysator verwendet.
4. Verfahren nach Anspruch 1, dadurch gekennzeichnet, daß man einen Eisensilikatzeolith-Katalysator verwendet.

**Claims**

1. A process for preparing diisobutene by dimerizing isobutene over a zeolite catalyst at elevated temperature, which comprises using a zeolite catalyst of the pentasil type containing from 0.5 to 7% by weight of bismuth and/or lead.
2. A process as claimed in claim 1, wherein an aluminosilicate zeolite catalyst is used.
3. A process as claimed in claim 1, wherein a borosilicate zeolite catalyst is used.
4. A process as claimed in claim 1, wherein an iron silicate zeolite catalyst is used.

**Revendications**

1. Procédé de préparation de diisobutène par dimérisation d'isobutène sur un catalyseur de zéolithe à températures élevées, caractérisé en ce qu'on utilise un catalyseur de type pentasil qui contient de 0,5 à 7% en poids de bismuth et/ou de plomb.

2. Procédé selon la revendication 1, caractérisé en ce qu'on utilise un catalyseur de zéolithe d'aluminosilicate.

3. Procédé selon la revendication 1, caractérisé en ce qu'on utilise un catalyseur de zéolithe de borosilicate.

4. Procédé selon la revendication 1, caractérisé en ce qu'on utilise un catalyseur de zéolithe de ferro-silicate.